# EUROPÄISCHE PATENTSCHRIFT

(11) **EP 1 313 476 B1**
(45) Veröffentlichungstag und Bekanntmachung des Hinweises auf die Patenterteilung: **25.10.2006**
(21) Anmeldenummer: 01962975.7
(22) Anmeldetag: 28.08.2001
(51) Int. Cl.: A61K 31/4741

(54) **SGK3 ALS DIAGNOSTISCHEs UND THERAPEUTISCHEs TARGET**
SGK3 USED AS DIAGNOSTIC AND THERAPEUTIC TARGET
SGK3 EN TANT QUE CIBLE DIAGNOSTIQUE ET THERAPEUTIQUE

(30) Priorität: 28.08.2000 DE 10042137
(43) Veröffentlichungstag der Anmeldung: 28.05.2003
(73) Patentinhaber: Lang, Florian Physiologisches Institut I, 72076 Tübingen (DE)
(72) Erfinder: LANG, Florian, 72076 Tübingen (DE); COHEN, Philip, Dept. of Biochemistry, Dundee DD1 5EH (GB); FRIEDRICH, Björn, Med. Klinik u. Poliklinik, 72076 Tübingen (DE)
(74) Vertreter: Mütschele, Thomas
(86) Internationale Anmeldenummer: PCT/EP2001/009890
(87) Internationale Veröffentlichungsnummer: WO 2002/017893

(56) Entgegenhaltungen:
- EP-A- 0 657 164
- EP-A- 0 889 127
- WO-A-99/02532
- WO-A-99/42592
- WO-A-99/61039
- DE-A- 19 917 990
- US-A- 5 137 912
- SCHILD L ET AL: "A MUTATION IN THE EPITHELIAL SODIUM CHANNEL CAUSING LIDDLE DISEASE INCREASES CHANNEL ACTIVITY IN THE XENOPUS LAEVIS OOCYTE EXPRESSION SYSTEM" PROCEEDINGS OF THE NATIONAL ACADEMY OF SCIENCES OF USA, NATIONAL ACADEMY OF SCIENCE. WASHINGTON, US, Bd. 92, 6. Juni 1995 (1995-06-06), Seiten 5699-5703, XP002900086 ISSN: 0027-8424
- DATABASE BIOSIS [Online] BIOSCIENCES INFORMATION SERVICE, PHILADELPHIA, PA, US; September 2000 (2000-09) FRIEDRICH BJOERN ET AL: "Serine/threonine kinases SGK2 and SGK3 both upregulate renal epithelial Na+-channel alpha,beta,gammarENaC." Database accession no. PREV200200223813 XP002198567 & JOURNAL OF THE AMERICAN SOCIETY OF NEPHROLOGY, Bd. 11, Nr. Program and Abstract Issue, September 2000 (2000-09), Seite 28A 33rd Annual Meeting of the American Society of Nephrology and the 2000 Renal Week;Toronto, Ontario, Canada; October 10-16, 2000, September, 2000 ISSN: 1046-6673
- DATABASE BIOSIS [Online] BIOSCIENCES INFORMATION SERVICE, PHILADELPHIA, PA, US; September 2000 (2000-09) WAGNER CARSTEN A ET AL: "The aldosterone induced kinase SGK1 stimulates the Kv1.3 potassium channel." Database accession no. PREV200200223871 XP002198568 & JOURNAL OF THE AMERICAN SOCIETY OF NEPHROLOGY, Bd. 11, Nr. Program and Abstract Issue, September 2000 (2000-09), Seite 38A 33rd Annual Meeting of the American Society of Nephrology and the 2000 Renal Week;Toronto, Ontario, Canada; October 10-16, 2000, September, 2000 ISSN: 1046-6673

## Beschreibung

Die vorliegende Erfindung betrifft die Verwendung eines Antikörpers zum diagnostischen Nachweis von sgk3 (serum and glucocorticoid dependent kinase 3) sowie die Verwendung von Staurosporin und/oder Chelerythrin und/oder SB 203580 (MG 377,4) oder SB 202190 (MG 331,3) zur Beeinflussung von sgk3 für die therapeutische Behandlung von Erkrankungen, die mit einer gestörten Aktivität von lonenkanälen, insbesondere Natrium- und/oder Kaliumkanälen in Zusammenhang stehen. Die sgk sind eine Serin/Threonin Proteinkinase Familie, die transkriptionell und post-transkriptionell reguliert werden.

Eine Vielzahl von externen Signalen, denen eine Zelle in ihrer Umwelt ausgesetzt ist, führen zu intrazellulären Phosphorylierungs-/Dephosphorylierungskaskaden, um eine schnelle und reversible Übertragung dieser Signale von der Plasmamembran und ihren Rezeptoren in das Zytoplasma und den Zellkern zu ermöglichen. Die Regulation einzelner Proteine, die an diesen Kaskaden beteiligt sind, ermöglich erst die hohe Spezifität und Flexibilität der Zellen, die es ihnen erlauben, sehr schnell auf extrazelluläre Signale zu reagieren. An diesen Regulationsprozessen sind insbesondere Kinasen beteiligt. Die serum- und glucocorticoidabhängige Kinase (sgk) wurde ursprünglich aus Rattenmammakarzinomazellen kloniert (Webster et al. 1993 a,b). Die humane Kinase hsgk wurde als zellvolumenreguliertes Gen aus Leberzellen kloniert (Waldegger et al. 1997). Es zeigte sich, daß die Rattenkinase (Chen et al. 1999, Näray-Fejes-Töth et al. 1999) den epithelialen Na⁺-Kanal (ENaC) stimuliert. Weiter wurde gezeigt, daß eine gesteigerte Aktivität des EnaC mit Hypertonie einhergeht (Warnock 1998).

Die hsgk wird auch im Gehirn exprimiert (Waldegger et al. 1997). Dort spielen die spannungsabhängigen K⁺-Kanäle Kv1.3 eine entscheidende Rolle bei der Regulation der neuronalen Erregbarkeit (Pongs 1992). Kv1.3 spielt ferner eine wichtige Rolle in der Regulation von Zellproliferation (Cahalan and Chandy, 1997) und apoptotischem Zelltod (Szabo et al., 1996, Lang et al., 1999). Kv1.3 ist ferner wichtig bei der Regulation der Lymphozytenproliferation und -funktion (Cahalan and Chandy, 1997). Kürzlich wurden zwei weitere Mitglieder der sgk-Familie kloniert, die sgk2 und sgk3 (Kobayashi et al., 1999). Wie die sgk1, werden auch die sgk2 und sgk3 durch Insulin und IGF1 über den P13 Kinase-Weg aktiviert. Eine weitere Charakterisierung und funktionelle Zuordnung beider neuer Kinasen hat bis jetzt aber nicht stattgefunden.

Die EP-A-0889127 offenbart die Verwendung von sgk2-Polypeptiden und -Polynukleotiden für das Design eines Protokolls zur Behandlung verschiedener Krankheiten. sgk3 ist nicht angesprochen.
Darüberhinaus offenbaren die WO 99/02532A und die EP-A-0657164 lediglich ganz allgemein die Verwendung von Staurosporinen in pharmazeutischen Formulierungen bei verschiedenen Krankheitsbildern. Die US-A-5137912 zitiert Chelerythrin als Medikament bei verschiedenen Krankheiten. SB203580 und Imidazole als Kinaseinhibitoren in pharmazeutischen Zusammensetzungen offenbart die WO 99/61039A. Die WO 99/42592A schließlich beschreibt die Kinaseinhibitoren SB203580 und SB202190.

Dementsprechend stellt sich die Erfindung die Aufgabe, die Kinase sgk3 für diagnostische und therapeutische Anwendungen nutzbar zu machen.

Überraschenderweise konnte in Experimenten mit der Zwei-Elektroden-Spannungsklemme gezeigt werden, daß Coexpression der hsgk2 oder der hsgk3 zu einer massiven Zunahme der Aktivität des epithelialen Na⁺- Kanals (ENaC) führt. Der ENaC spielt eine entscheidende Rolle bei der renalen Na⁺-Ausscheidung, die wiederum den Blutdruck beeinflußt. Die Kinase sgk3 wird auch im Gehirn exprimiert. In Experimenten mit der Zwei-Elektroden-Spannungsklemme konnte auch gezeigt werden, daß die Coexpression der hsgk2 oder der hsgk3 zu einer Zunahme der Aktivität des K⁺-Kanales Kv1.3 führt. Da die Aktivierung von K⁺-Kanälen zu einer Herabsetzung der neuronalen Erregbarkeit führt, zeigen die gefundenen funktionellen Daten, daß die Wirkungen der sgk3 geeignet sind, die Erregbarkeit von Neuronen zu reduzieren. Eine gestörte Expression oder Funktion der sgk3 kann somit Ursache für das Auftreten epileptischer Anfälle sein. Umgekehrt ist der Schluss berechtigt, daß Stimulatoren der Expression oder Aktivität der sgk3, welche die Bluthirnschranke überschreiten, bei epileptischen Anfällen mit Erfolg eingesetzt werden können. Schließlich wurde gefunden, daß der im Herzen exprimierte K⁺-Kanal minK durch die sgk1, sgk2, sgk3 aktiviert wird. Damit spielen diese Kinasen eine Rolle bei der Regulation der kardialen Erregbarkeit.

Demzufolge wird die erfindungsgemäße Aufgabe durch den Gegenstand der unabhängigen Ansprüche 1 und 2 gelöst. Bevorzugte Ausführungen sind in den abhängigen Ansprüchen 3 bis 9 genannt. Der Inhalt aller dieser Ansprüche wird hiermit durch Bezugnahme zum Inhalt der Beschreibung gemacht.

Erfindungsgemäß wird mindestens ein Antikörper zum Nachweis der Expression und/oder der Funktion von sgk3 in eukaryotischen Zellen verwendet werden. Damit ist insbesondere auch eine Diagnose von Erkrankungen, die mit einer gestörten Aktivität von Ionenkanälen, wie z. B. Natrium- und Kaliumkanälen in Zusammenhang stehen, möglich. Bei diesem Antikörper könnte es sich z. B. um einen Antikörper handeln, der gegen sgk2 oder sgk3 gerichtet ist, und in einem dem Fachmann bekannten Nachweisverfahren, wie z.B. ELISA (enzyme-linked immuno sorbent assay) eingesetzt werden kann. Bei solchen Immunoassays wird der gegen das zu bestimmende Antigen (sgk2 und sgk3) gerichtete spezifische Antikörper (bzw. bei Antikörperbestimmungen homologe Testantigene) an eine Trägersubstanz (z.B. Zellulose, Polystyrol) gebunden, an der sich nach der Inkubation mit der Probe Immunkomplexe bilden. In einem nachfolgenden Schritt wird diesen Immunkomplexen ein markierter Antikörper zugeführt. Durch Zugabe eines chromogenen Substrates zum Reaktionsansatz können die Immunkomplexgebundenen Enzym-Substrat-Komplexe sichtbar gemacht bzw. die Antigenkonzentration in der Probe über eine photometrische Bestimmung der Immunkomplex-gebundenenen Markerenzyme durch Vergleich mit Standards bekannter Enzymaktivität ermittelt werden. Weitere Substanzen, die für den diagnostischen Nachweis verwendet werden können, sind sogenannte Oligonukleotide, die mit Hilfe der sogenannten Polymerase-Kettenreaktion (PCR) geeignet sind, über ein molekulargenetisches Verfahren, bei dem selektiv bestimmte DNA-Abschnitte amplifiziert werden, einen quantitativen Nachweis von sgk3 zu erbringen. Weitere Verfahren, wie ein bekanntes Zielprotein quantitativ nachgewiesen werden kann, sind dem Fachmann geläufig.

Erfindungsgemäß wird ein Wirkstoff, nämlich Staurosporin und/oder Chelerythrin und/oder SB 203580 (MG 377,4) oder SB 202190 (MG 331,3), für die Beeinflussung, insbesondere die Inhibierung oder Aktivierung der Expression und/oder Funktion von sgk3 in eukaryotischen Zellen, zur Behandlung von Erkrankungen, die mit einer gestörten Aktivität von lonenkanälen, insbesondere Natrium und/oder Kalium-Kanälen in Zusammenhang stehen, beansprucht. Da es sich bei sgk3 um eine Kinase handelt, kommen als Kinase-Inhibitoren bekannte Substanzen wie die erfindungsgemäß verwendeten Staurosporin, Chelerythrin etc., aber auch andere Substanzen in Frage. Dem Fachmann sind solche Inhibitoren bekannt, und man kann sie teilweise kommerziell bei Firmen wie Sigma oder Merck beziehen. Als Aktivatoren können z. B. gentechnisch veränderte Mutanten von sgk3 genutzt werden.

Erfindungsgemäß kann es sich bei dem Ionenkanal um einen NatriumKanal des ENaC-Subtypes handeln. Dabei wird durch die Inhibierung bzw. Aktivierung von sgk3 vorzugsweise der Na⁺-Transport durch diesen Kanal beeinflußt, der seinerseits beispielsweise den Blutdruck beeinflußt. Durch die Überexpression oder Überaktivität der sgk3 kommt es zu einer renalen Retention von Na⁺ und auf diese Weise zur Entwicklung von Hypertonie. Es ist somit möglich, durch Aktivierung bzw. Inaktivierung der entsprechenden Kinasen den Blutdruck zu regulieren.

In einer bevorzugten Ausführungsform handelt es sich bei dem lonenkanal um einen Kalium-Kanal des Kv1.3-Subtypes. Die Beeinflussung, insbesondere die Inhibierung oder Aktivierung von sgk3 beeinflußt vorzugsweise den K⁺-Transport durch den Kalium-Kanal vom Kv1.3-Subtyp. Bei anderen bevorzugten Ausführungsformen handelt es sich bei dem lonenkanal um einen Kalium-Kanal des minK-Subtypes, wobei hier eine Beeinflussung von sgk1, sgk2 und/oder sgk3 den K⁺-Transport durch den Kalium-Kanal vom minK-Subtyp beeinflußt.

In einer bevorzugten Ausführungsform der Erfindung ist der Wirkstoff, nämlich Staurosporin und/oder Chelerythrin und/oder SB 203580 (MG 377,4) oder SB 202190 (MG 331,3), gegen sgk3 selbst gerichtet. Es kann sich dann bei den Wirkstoffen um Antisensesequenzen, sogenannte kinase deficient mutants, oder auch um Kinaseinhibitoren, wie die erfindungsgemäß verwendeten Staurosporin und/oder Chelerythrin bzw. deren Analoga handeln. Auch sogenannte "small molecular compounds" bzw. Polynukleotide, welche für ein Peptid kodieren, welches die Expression von sgk3 beeinflußt, können verwendet werden.

In einer anderen bevorzugten Ausführungsform der Erfindung ist der Wirkstoff, nämlich Staurosporin und/oder Chelerythrin und/oder SB 203580 (MG 377,4) oder SB 202190 (MG 331,3), gegen Aktivatoren, Inhibitoren, Regulatoren und/oder biologische Vorläufer von sgk3 gerichtet. Diese Aktivatoren, Inhibitoren, Regulatoren und/oder biologische Vorläufer könnten up- und downstream liegende Mitglieder der sgk-Signaltransduktionskaskade, Transkriptionsfaktoren, die für den Expressionslevel von sgk3 verantwortlich sind, aber auch bis jetzt unbekannte Moleküle sein, die durch den Wirkstoff, nämlich Staurosporin und/oder Chelerythrin und/oder SB 203580 (MG 377,4) oder SB 202190 (MG 331,3), beeinflußt werden und an der Expression und/oder Funktion von sgk3 beteiligt sind.

Bei der Erfindung ist es möglich, bekannte sowie noch unbekannte Wirkstoffe zu verwenden. In einer besonders bevorzugten Ausführungsform ist der Wirkstoff eine sogenannte "small molecular compound", insbesondere eine solche mit einem Molekulargewicht (MG) < 1000. Bei dieser small molecular compound kann es sich auch um Kinase-Inhibitoren, wie die erfindungsgemäß verwendeten Imidazol-Derivate SB 203580 (MG 377,4) oder auch SB 202190 (MG 331,3) handeln, die beide bekannte Inhibitoren der Kinase-Expression sind und von Calbiochem, San Diego, CA, USA, kommerziell vertrieben werden.

Die Erfindung kann genutzt werden, um alle Formen von Erkrankungen, die mit einer gestörten Aktivität von Natrium- und/ oder Kaliumkanälen in Zusammenhang stehen, zu behandeln. Insbesondere ist hierbei an die arterielle Hypertonie, sowie ein dem sogenannten Liddle-Syndrom eine seltene genetisch bedingte Überaktivität des ENaC und somit eine Erkrankung mit massiver Steigerung des Blutdruckes entsprechendes Krankheitsbild zu denken.

Die durch die erfindungsgemäße Verwendung behandelbaren Erkrankungen, die mit einer gestörten Aktivität von Kaliumkanälen, insbesondere des Kv1.3- bzw. des minK-Subtypes verbunden sind, umfassen nach derzeitiger Kenntnis die Erkrankungen Epilepsie, Neurodegeneration, Autoimmunerkrankungen sowie Immundefizienz. Störungen des minK-Kanales sind insbesondere Ursache von Herzrhythmusstörungen.

Die Erfindung betrifft auch einen Diagnosekit. Dieser umfaßt mindestens einen Antikörper der zum Nachweis von Expression und/oder Funktion von sgk3 geeignet ist, zur Diagnose von Erkrankungen, die mit einer gestörten Aktivität von lonenkanälen, insbesondere Natrium- und/oder Kaliumkanälen in Zusammenhang stehen. Weiterhin können mit einem solchen Kit Erkrankungen diagnostiziert werden, die mit einer Über- oder Unterexpression bzw. -funktion von sgk3 verbunden sind. Gezielt können solche Diagnostika in einem Diagnosekit eingesetzt werden, um Erkrankungen wie arterielle Hypertonie, Liddle-Syndrom, Epilepsie, Neurodegeneration, Autoimmunerkrankungen und Immundefizienz nachzuweisen. Auch hierbei erfolgt der Nachweis der Erkrankungen über den Nachweis einer gestörten Expression und/oder Funktion von sgk3.

Die Erfindung umfaßt ferner eine pharmazeutische Zusammensetzung, die mindestens einen Wirkstoff, nämlich Staurosporin und/oder Chelerythrin und/oder SB 203580 (MG 377,4) oder SB 202190 (MG 331,3), enthält, der die Expression und/oder Funktion von sgk3 beeinflußt, insbesondere inhibiert oder aktiviert, und vorzugsweise, ggf. einen pharmazeutischen Träger. Dabei kann es sich bei dem Wirkstoff um einen Kinase-Inhibitor, wie die bereits weiter oben erwähnten und erfindungsgemäß verwendeten Inhibitoren Staurosporin, Chelerythrin, SB 203580 und SB 202190 bzw. deren Analoga, aber auch andere Substanzen, handeln. Bei dem Wirkstoff kann es sich ferner um ein Polynukleotid, welches ein Peptid, vorzugsweise ein Polypeptid kodiert, handeln, wobei dieses Peptid die Expression von sgk3 beeinflußt, vorzugsweise inhibiert oder aktiviert. Dieses Polypeptid könnte zum Beispiel ein sogenannter "kinase deficient mutant" sein. Bei dem Wirkstoff kann es sich ferner um eine sogenannte "small molecular compound" handeln, vorzugsweise um small molecular compounds mit einem Molekulargewicht (MG) < 1000. Schließlich kann es sich bei dem Wirkstoff auch um eine sogenannte Antisensesequenz handeln, d.h. eine Sequenz, die in der Lage ist, mit der mRNA eine Doppelstrangduplex auszubilden, und dadurch die Translation in ein Polypeptid zu verhindern. Auch könnte die Sequenz von sgk3 selbst genutzt werden, um eine Überexpression dieser Kinasen zu erzielen, z. B. durch Einbau von Vektoren mit starken Promotoren. Zu weiteren Merkmalen einer solchen Zusammensetzung wird auf den entsprechenden bisherigen Text der Beschreibung Bezug genommen.

Schließlich umfaßt die Erfindung eine pharmazeutische Zusammensetzung, die eine wirksame Menge mindestens eines Wirkstoffes, nämlich von Staurosporin und/oder Chelerythrin und/oder SB 203580 (MG 377,4) oder SB 202190 (MG 331,3), aufweist, der die Expression und/oder Funktion von Aktivatoren, Inhibitoren, Regulatoren und/oder biologischen Vorläufern von sgk3 beeinflußt, insbesondere inhibiert oder aktiviert. Vorzugsweise kann diese pharmazeutische Zusammensetzung ggf. auch einen pharmazeutischen Träger enthalten. Diese Aktivatoren, Inhibitoren, Regulatoren und/oder biologische Vorläufer von sgk3 können z.B. weitere Kinasen sein, die an der Regulation der Aktivität von sgk3 beteiligt sind, Transkriptionsfaktoren, die für den Expressionslevel von sgk3 verantwortlich sind, sowie weitere bekannte oder bis jetzt unbekannte Mitglieder der sgk3 Signaltransduktionskaskade. Auch Polynukleotide, die ein Peptid kodieren, welches die Expression von Aktivatoren, Inhibitoren, Regulatoren und/oder biologischen Vorläufern von sgk3 beeinflußt, vorzugsweise inhibiert oder aktiviert, können in solchen Zusammensetzungen enthalten sein. Auch sogenannte small molecular compounds, die vorzugsweise ein Molekulargewicht (MG) von < 1000 haben, und die gegen Aktivatoren, Inhibitoren, Regulatoren und/oder biologische Vorläufer von sgk3 gerichtet sind, und dabei die Expression bzw. Funktion dieser Kinasen inhibieren oder aktivieren, sind einsetzbar.

Die bestehenden Merkmale und weitere Merkmale der Erfindung ergeben sich aus der nachfolgenden Beschreibung von bevorzugten Ausführungsformen in Verbindung mit den Unteransprüchen und den Figuren. Hierbei können die einzelnen Merkmale jeweils für sich oder zu mehreren in Kombination miteinander verwirklicht sein.

In den Abbildungen zeigen:
- Figur 1:: Stimulation des Na⁺-Kanales rENaC durch die hsgk2 und hsgk3.
- Figur 2:: Stimulation des K⁺-Kanales Kv1.3 durch die hsgk2 und hsgk3.
- Figur 3:: Wirkung einer Hemmung des K⁺-Kanales Kv1.3 auf das Überleben von HEK-Zellen (human embryonic kidney cells).

### Material und Methoden

Die Dissektion von Xenopus laevis, die Gewinnung und Behandlung der Oozyten wurde im Detail früher beschrieben (Busch et al., 1992). Die Oozyten wurden je mit 1 ng cRNA von α, β, γ ENaC und des Kv1.3 bzw. des minK mit oder ohne gleichzeitige Injektion der Kinasen hsgk1, hsgk2 und hsgk3 injiziert. Zweielektroden-Spannungs- und Stromklemme-Experimente konnten 2 bis 4 Tage nach Injektion durchgeführt werden. Na⁺-Ströme (ENaC) und K⁺-Ströme (Kv1.3, minK) wurden bei 10 Hz gefiltert und mit einem Schreiber aufgezeichnet. Die Experimente wurden normalerweise am zweiten Tag nach cRNA Injektion durchgeführt. Die Badlösung enthielt: 96 mM NaCl, 2mM KCI, 1.8 mM CaCl₂, 1 mM MgCl₂, und 5 mM HEPES bei pH 7.5 und bei einem Haltepotential von -80 mV. In allen Experimenten wurde der pH durch Titration mit HCl oder NaOH eingestellt. Die Flußrate der Badflüssigkeit wurde auf 20 ml/min eingestellt, wodurch ein kompletter Lösungswechsel in der Meßkammer innerhalb von 10 bis 15 Sekunden gewährleistet wurde. Alle Daten werden in Form von arithmetischen Mittetwerten ± SEM angegeben.

### Ergebnisse

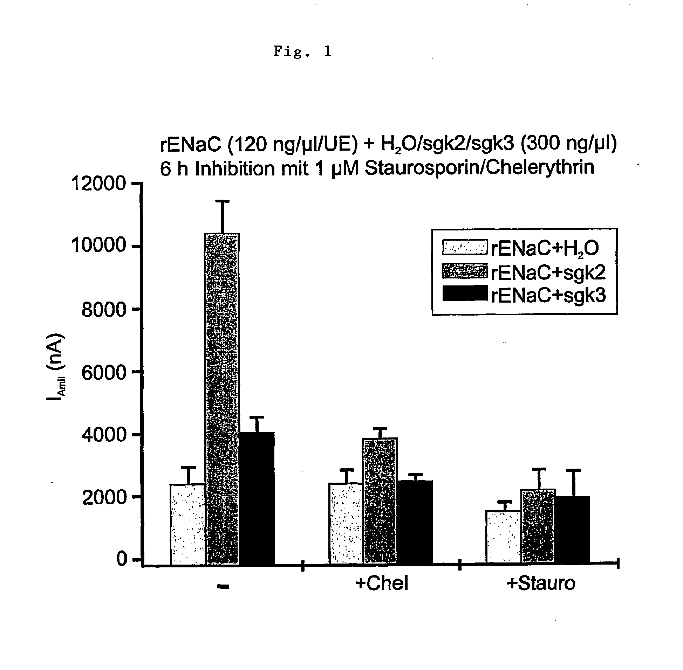

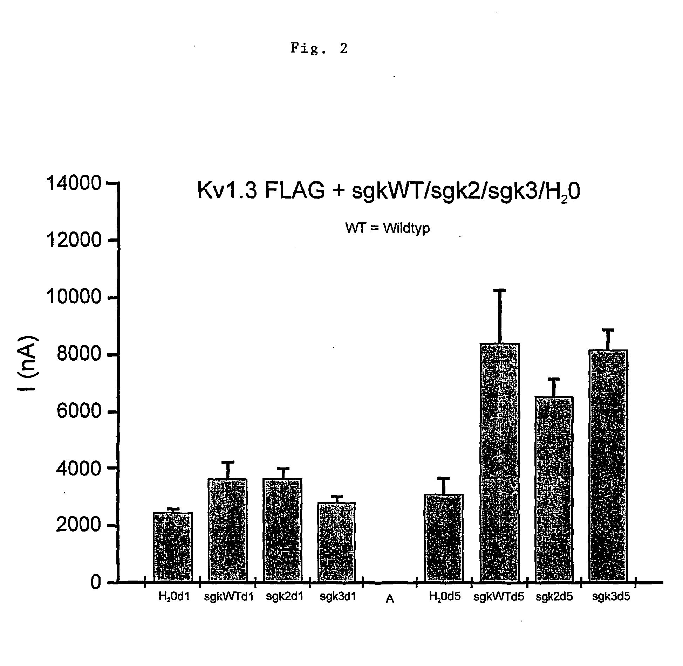

Um die Wirkungen von hsgk1, hsgk2 oder hsgk3 zu untersuchen, wurde die mRNA der jeweiligen Kinase zusammen mit der mRNA des epithelialen Na⁺-Kanales α, β, γ ENaC oder des spannungsabhängigen K⁺-Kanales Kv1.3 bzw. des minK-Kanales in Xenopus-Oozyten injiziert und danach wurde der Amilorid-sensitive Na⁺-Strom (I_{Na}) und der spannungs-aktivierte K⁺-Strom (I_{K}) bestimmt. Wie in der folgenden Tabelle 1 sowie in Abb. 1 und 2 gezeigt wird, stimulieren sowohl hsgk2 als auch hsgk3 sowohl die ENaC- als auch die Kv1.3-Aktivität. Die hsgk1 stimuliert die minK-Aktivität (Tabelle 1). Die stimulierende Wirkung wurde durch die Proteinkinase-Inhibitoren Staurosporin und Chelerythrin völlig unterbunden.

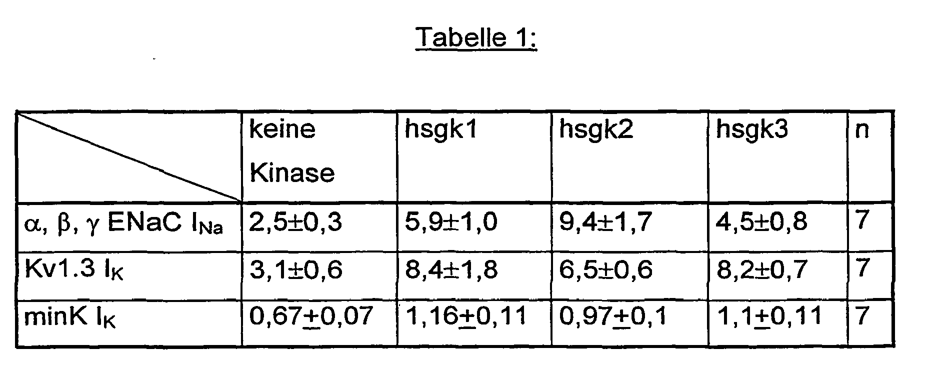

Tabelle 1: Na⁺ (I_{Na}) und K⁺ (l_{K}) -Ströme (µA) in Oozyten, die mit Wasser (n.i.), mit α, β, γ ENaC, mit Kv1.3 oder mit minK mit oder ohne hsgk1, hsgk2 oder hsgk3 injiziert wurden.

### Experiment 1

Nach Injektion der mRNA von hsgk2 und hsgk3 konnte gezeigt werden (siehe Figur 1), daß der Amilorid-hemmbare Strom durch den Na⁺-Kanal rENaC (I_{Amil}) durch die Coexpression mit hsgk2 und hsgk3 signifikant zunimmt. Die Kinasehemmer Staurosporin und Chelerythrin hemmen die Aktivierung des Na+-Kanales (Figur 1). Da die stimulierende Wirkung der hsgk2 und hsgk3 auf den ENaC-Kanal durch die Kinasehemmstoffe Staurosporin und Chelerythrin unterbunden werden kann, kann a) der diagnostische Nachweis einer gestörten Expression oder Funktion der sgk2 oder der sgk3 eine wichtige Maßnahme sein, die Ursache beispielsweise einer vorliegenden Hypertonie aufzudecken und b) der Einsatz der Hemmstoffe der sgk2 und sgk3, wie Staurosporin, Chelerythrin oder weitere Kinasehemmer, bei der Therapie der oben genannten Erkrankung möglich sein.

### Experiment 2

Nach Injektion der mRNA von hsgk1, hsgk2 oder hsgk3 zusammen mit der mRNA der K⁺-Kanäle Kv1.3 oder minK konnte gezeigt werden, daß der Strom durch diese beiden Kanäle (I) gesteigert werden kann (Tabelle 1). Abbildung 2 zeigt die Ergebnisse dieser Experimente nach Injektion der mRNA von hsgk2 und hsgk3 zusammen mit der mRNA von Kv1.3 am ersten Tag (d1, linke Säulen) und am fünften Tag (d5, rechte Säulen). Da die Aktivierung von K⁺-Kanälen zu einer Herabsetzung der neuronalen Erregbarkeit führt, zeigen diese funktionellen Daten, daß die Wirkungen der im Gehirn exprimierten hsgk3 geeignet sind, die Erregbarkeit von Neuronen zu reduzieren. Eine gestörte Expression oder Funktion der sgk3 kann somit Ursache für das Auftreten epileptischer Anfälle sein. Umgekehrt können Stimulatoren der Expression oder Aktivität der sgk3, welche die Bluthirnschranke überschreiten, bei epileptischen Anfällen eingesetzt werden. Gleiche Überlegungen gelten bei Stimulation bzw. Inhibierung der Kinasen, insbesondere der hsgk1, zur Beeinflussung gestörter Erregbarkeit des Herzens.

### Experiment 3

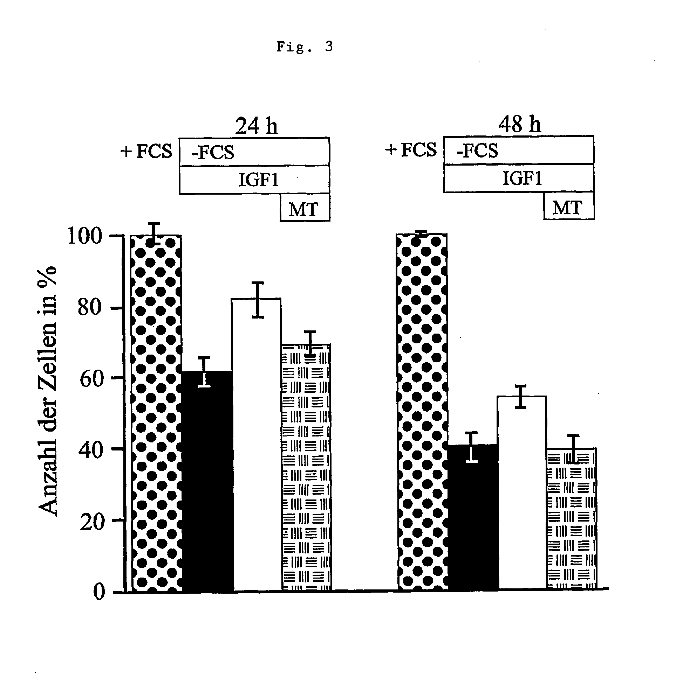

Gemäss Figur 3 führt der Entzug von fötalem Kälberserum (FCS) in HEK-Zellen (human embryonic kidney cells; Lewis, M.L. et al. 1984, Phillips, S.G. et al. 1982) zur Abnahme der Zellzahl durch Absterben von Zellen (schwarze Säulen im Vergleich zu den gepunkteten Säulen), wobei die Situation jeweils nach 24 und 48 Stunden dargestellt ist. Die Abnahme wird durch den insulinähnlichen Faktor (IGF1) gemindert (weiße Säulen). Die Wirkung von IGF1 wird durch gleichzeitige Hemmung von K⁺-Kanälen mit Margatoxin (MT) aufgehoben (schraffierte Säulen). Diese Daten zeigen, daß der insulinähnliche Wachstumsfaktor (Insulin-like growth factor IGF1) seine zelltodhemmende Wirkung verliert, wenn gleichzeitig K⁺-Kanäle gehemmt werden. Die sgk2 und/oder sgk3 vermittelte Aktivierung des Kv1.3-Kanales wirkt demnach antiapoptotisch, und ein Mangel an sgk2 und sgk3 Wirkung würde demnach zu gesteigertem Zelltod, wie etwa bei Neurodegeneration, führen. Umgekehrt können Aktivatoren der sgk2 und sgk3 zur Verhinderung von apoptotischem Zelltod bei Neurodegeneration eingesetzt werden. Da Kv1.3 zudem eine wesentliche Rolle bei der Regulation der Lymphozytenproliferation und -funktion spielt, können Hemmstoffe bzw. Aktivatoren der Kinase zur Beeinflussung des Immunsystems z.B. bei Autoimmunerkrankung bzw. bei Immundefizienz eingesetzt werden.

### Literatur:

Busch AE, Kavanaugh MP, Varnum MD, Adelman JP, North RA. Regulation by second messengers of the slowly activating, voltage-dependent potassium current expressed in Xenopus oocytes. J. Physiol. Lond. 450: 491-502, 1992.
Cahalan MD, Chandy KG. Ion channels in the immune system as targets for immunosuppression. Cur. Opin. Biotech. 8 (6): 749-756, 1997.
Chen SY, Bhargava A, Mastroberardino L, Meijer OC, Wang J, Buse P, Firestone GL, Verrey F, Pearce D. Epithelial sodium channel regulated by aldosterone-induced protein sgk. Proc Natl Acad Sci USA 96: 2514-2519,1999.
Kobayashi T, Deak M, Morrice N, Cohen P. Characterization of the structure and regulation of two novel isoforms of serum- and glucocorticoid-induced protein kinase. Biochem. J. 344: 189-197, 1.999.
Lang F, Szabo I, Lepple-Wienhues A, Siemen D, Gulbins E. Physiology of receptor mediated lymphocyte apoptosis. News Physiol. Sci. 14: 194-200, 1999.
Lewis, ML, Morrison, DR, Mieszkuc, BJ, Fessler, DL. Problems in the bioassay of products from cultured HEK cells: plasminogen activator. Adv Exp Med Biol. 172: 241-267, 1984.
Naray-Fejes-Toth A, Canessa C, Cleaveland ES, Aldrich G, Fejes-Toth G. Sgk is an aldosterone-induced kinase in the renal collecting duct. Effects on epithelial Na+ channels. J. Biol. Chem. 274: 16973-16978, 1999.
Phillips, SG, Lui, SL, Phillips, DM. Binding of epithelial cells to lectincoated surfaces. In Vitro 18: 727-738, 1982.
Pongs O. Molecular biology of voltage-dependent potassium channels. Physiol Rev. 72: S69-S88, 1992.
Szabo I, Gulbins E, Apfel H, Zhan X, Barth P, Busch AE, Schlottmann K, Pongs O, Lang F. Tyrosine phosphorylation-dependent suppression of a voltage-gated K+-channel in T lymphocytes upon Fas stimulation. J. Biol. Chem. 271: 20465-20469, 1996.
Waldegger S, Barth P, Raber G, Lang F. Cloning and characterization of a putative human serine/threonine protein kinase transcriptionally modified during anisotonic and isotonic alterations of cell volume. Proc. Natl. Acad. Sci. USA 94: 4440-4445, 1997.
   Warnock DG. Liddle syndrome: An autosomal dominant form of human hypertension. Kidney Ind. 53 (1): 18-24, 1998.
Webster MK, Goya L, Firestone GL. Immediate-early transcriptional regulation and rapid mRNA turnover of a putative serine/threonine protein kinase. J. Biol. Chem. 268 (16): 11482-11485, 1993a.
Webster MK, Goya L, Ge Y, Maiyar AC, Firestone GL. Characterization of sgk, a novel member of the serine/threonine protein kinase gene family which is transcriptionally induced by glucocorticoids and serum. Mol. Cell. Biol. 13 (4); 2031-2040, 1993b.

## Patentansprüche

1. Verwendung eines Antikörpers zum Nachweis der Expression und/oder Funktion von sgk3 in eukaryotischen Zellen, zur Diagnose von Erkrankungen, die mit einer gestörten Aktivität von lonenkanälen in Zusammenhang stehen.

2. Verwendung von Staurosporin oder Chelerythrin oder SB 203580 (MG 377,4) oder SB 202190 (MG 331,3) zur Beeinflussung der Expression und/oder Funktion von sgk3 in eukaryotischen Zellen, zur Behandlung von Erkrankungen, die mit einer gestörten Aktivität von Ionenkanälen in Zusammenhang stehen.

3. Verwendung nach Anspruch 2, **dadurch gekennzeichnet, daß** die Beeinflussung von sgk3, eine Beeinflussung und/oder Kontrolle der Na⁺-Ausscheidung und/oder K⁺-Ausscheidung zur Folge hat.

4. Verwendung nach einem der vorhergehenden Ansprüche, **dadurch gekennzeichnet, dass** es sich bei dem lonenkanal um einen Natriumkanal oder um einen Kaliumkanal handelt.

5. Verwendung nach einem der vorhergehenden Ansprüche, **dadurch gekennzeichnet, daß** es sich bei dem Ionenkanal um einen Natriumkanal des ENaC-Subtyps handelt.

6. Verwendung nach einem der vorhergehenden Ansprüche, **dadurch gekennzeichnet, daß** es sich bei dem lonenkanal um einen Kaliumkanal des Kv1.3-Subtyps handelt.

7. Verwendung nach einem der vorhergehenden Ansprüche, **dadurch gekennzeichnet, daß** es sich bei den Erkrankungen um arterielle Hypertonie oder um ein dem Liddle-Syndrom entsprechendes Krankheitsbild handelt.

8. Verwendung nach einem der vorhergehenden Ansprüche, **dadurch gekennzeichnet, daß** es sich bei den Erkrankungen um Epilepsie, Neurodegeneration, Autoimmunerkrankungen oder Immundefizienz handelt.

9. Verwendung nach einem der Ansprüche 1, 5, 6, 7 oder 8 in einem Diagnosekit.

## Claims

1. Use of an antibody for detecting the expression and/or function of sgk3 in eukaryotic cells for the diagnosis of diseases which are associated with an impaired activity of ion channels.

2. Use of staurosporin or chelerythrin or SB 203580 (MW 377.4) or SB 202190 (MW 331.3) for influencing the expression and/or function of sgk3 in eukaryotic cells for the treatment of diseases which are associated with an impaired activity of ion channels.

3. Use according to Claim 2, **characterized in that** influencing sgk3 results in influencing and/or controlling the Na⁺ excretion and/or K⁺ excretion.

4. Use according to one of the preceding claims, **characterized in that** the ion channel is a sodium channel or a potassium channel.

5. Use according to one of the preceding claims, **characterized in that** the ion channel is a sodium channel of the ENaC subtype.

6. Use according to one of the preceding claims, **characterized in that** the ion channel is a potassium channel of the Kv1.3 subtype.

7. Use according to one of the preceding claims, **characterized in that** the diseases are arterial hypertension or a clinical picture which corresponds to Liddle's syndrome.

8. Use according to one of the preceding claims, **characterized in that** the diseases are epilepsy, neurodegeneration, autoimmune diseases or immune deficiency.

9. Use according to one of Claims 1, 5, 6, 7 or 8 in a diagnostic kit.

## Revendications

1. Utilisation d'un anticorps pour déceler l'expression et/ou la fonction de sgk3 dans des cellules eucaryotes, pour le diagnostic de maladies qui sont en rapport avec une activité perturbée des canaux ioniques.

2. Utilisation de staurosporine ou de chélérythrine ou de SB 203580 (MG 377,4) ou de SB 202190 (MG 331,3) pour influencer l'expression et/ou la fonction de sgk3 dans des cellules eucaryotes, en vue du traitement de maladies qui sont en rapport avec une activité perturbée de canaux ioniques.

3. Utilisation suivant la revendication 2, **caractérisée en ce que** l'influence sur sgk3 a pour conséquence une influence et/ou un contrôle de l'excrétion de Na⁺ et/ou de l'excrétion de K⁺.

4. Utilisation suivant l'une des revendications précédentes, **caractérisée en ce que**, pour ce qui concerne le canal ionique, il s'agit d'un canal à sodium ou d'un canal à potassium.

5. Utilisation suivant l'une des revendications précédentes, **caractérisée en ce que**, pour ce qui concerne le canal ionique, il s'agit d'un canal à sodium du sous-type ENaC.

6. Utilisation suivant l'une des revendications précédentes, **caractérisée en ce que**, pour ce qui concerne le canal ionique, il s'agit d'un canal à potassium du sous-type Kv1.3.

7. Utilisation suivant l'une des revendications précédentes, **caractérisée en ce que**, pour ce qui concerne les maladies, il s'agit d'une hypertonie artérielle ou des symptômes d'une maladie correspondant au syndrome de Liddle.

8. Utilisation suivant l'une des revendications précédentes, **caractérisée en ce que**, pour ce qui concerne les maladies, il s'agit d'épilepsie, de neurodégénération, de maladies auto-immunes ou de déficience immunitaire.

9. Utilisation suivant l'une des revendications 1, 5, 6, 7 ou 8 dans une trousse de diagnostic.
